# EUROPEAN PATENT SPECIFICATION

(11) **EP 3 616 627 B1**
(45) Date of publication and mention of the grant of the patent: **26.06.2024**
(21) Application number: 19195235.7
(22) Date of filing: 28.10.2016
(51) Int. Cl.: A61B 17/072

(54) **SURGICAL STAPLER BUTTRESS ASSEMBLY WITH MULTI-LAYER ADHESIVE**
CHIRURGISCHE KLAMMERVERSTEIFUNGSANORDNUNGEN MIT MEHRSCHICHTIGEN KLEBERSCHICHTEN
ENSEMBLE RENFORT D'AGRAFEUSE CHIRURGICALE A ADHESIF MULTICOUCHE

(30) Priority: 29.10.2015 US 201514926160
(43) Date of publication of application: 04.03.2020
(62) Divisional of application: 16196410.1
(73) Proprietor: Ethicon LLC, Guaynabo 00969 (PR)
(72) Inventor: SHELTON, IV, Frederick E., Cincinnati, OH 45242 (US); HARRIS, Jason L., Cincinnati, OH 45242 (US); VENDELY, Michael J., Cincinnati, OH 45242 (US)
(74) Representative: Carpmaels & Ransford LLP

(56) References cited:
- EP-A1- 1 064 883
- EP-A2- 2 008 595
- EP-A2- 3 072 453
- EP-A2- 3 072 455
- US-A1- 2013 214 030

## Description

### BACKGROUND

EP 3 072 453 A2 and EP 3 072 455 A2 (both prior art under Article 54(3) EPC) disclose a buttress assembly comprising a buttress body, an upper adhesive layer, and a lower adhesive layer. EP 1 064 883 A1 discloses a foam buttress which may be processed to prevent wicking of an adhesive layer into the interior of the foam.

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through the cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasonic vibration, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; and U.S. Pat. No. 8,453,914, entitled "Motor-Driven Surgical Cutting Instrument with Electric Actuator Directional Control Assembly," issued June 4, 2013. The disclosure of each of the above-cited U.S. Patents is referenced herein.

While the surgical staplers referred to above are described as being used in endoscopic procedures, it should be understood that such surgical staplers may also be used in open procedures and/or other non-endoscopic procedures. By way of example only, a surgical stapler may be inserted through a thoracotomy, and thereby between a patient's ribs, to reach one or more organs in a thoracic surgical procedure that does not use a trocar as a conduit for the stapler. Such procedures may include the use of the stapler to sever and close a vessel leading to a lung. For instance, the vessels leading to an organ may be severed and closed by a stapler before removal of the organ from the thoracic cavity. Of course, surgical staplers may be used in various other settings and procedures.

Examples of surgical staplers that may be particularly suited for use through a thoracotomy are disclosed in U.S. Patent Pub. No. 2014/0243801, entitled "Surgical Instrument End Effector Articulation Drive with Pinion and Opposing Racks," published August 28, 2014; U.S. Patent Pub. No. 2014/0239041, entitled "Lockout Feature for Movable Cutting Member of Surgical Instrument," published August 28, 2014; U.S. Patent Pub. No. 2014/0239042, entitled "Integrated Tissue Positioning and Jaw Alignment Features for Surgical Stapler," published August 28, 2014; U.S. Patent Pub. No. 2014/0239036, entitled "Jaw Closure Feature for End Effector of Surgical Instrument," published August 28, 2014; U.S. Patent Pub. No. 2014/0239040, entitled "Surgical Instrument with Articulation Lock having a Detenting Binary Spring," published August 28, 2014; U.S. Patent Pub. No. 2014/0239043, entitled "Distal Tip Features for End Effector of Surgical Instrument," published August 28, 2014; U.S. Patent Pub. No. 2014/0239037, entitled "Staple Forming Features for Surgical Stapling Instrument," published August 28, 2014; U.S. Patent Pub. No. 2014/0239038, entitled "Surgical Instrument with Multi-Diameter Shaft," published August 28, 2014; and U.S. Patent Pub. No. 2014/0239044, entitled "Installation Features for Surgical Instrument End Effector Cartridge," published August 28, 2014. The disclosure of each of the above-cited U.S. Patent Publications is referenced herein.

Additional surgical stapling instruments are disclosed in U.S. Pat. No. 8,801,735, entitled "Surgical Circular Stapler with Tissue Retention Arrangements," issued August 12, 2014; U.S. Pat. No. 8,141,762, entitled "Surgical Stapler Comprising a Staple Pocket," issued March 27, 2012; U.S. Pat. No. 8,371,491, entitled "Surgical End Effector Having Buttress Retention Features," issued February 12, 2013; U.S. Pub. No. 2014/0263563, entitled "Method and Apparatus for Sealing End-to-End Anastomosis" published September 18, 2014; U.S. Pub. No. 2014/0246473, entitled "Rotary Powered Surgical Instruments with Multiple Degrees of Freedom," published September 4, 2014; U.S. Pub. No. 2013/0206813, entitled "Linear Stapler," published August 15, 2013; U.S. Pub. No. 2008/0169328, entitled "Buttress Material for Use with a Surgical Stapler," published July 17, 2008; U.S. Pat. App. No. 14/300,804, entitled "Woven and Fibrous Materials for Reinforcing a Staple Line," filed June 10, 2014; U.S. Pat. App. No. 14/300,811, entitled "Devices and Methods for Sealing Staples in Tissue"; and U.S. Pat. App. No. 14/498,070, entitled "Radically Expandable Staple Line" filed September 26, 2014. The disclosure of each of the above-cited U.S. Patents, U.S. Patent Publications, and U.S. Patent Applications is referenced herein.

In some instances, it may be desirable to equip a surgical stapling instrument with a buttress material to reinforce the mechanical fastening of tissue provided by staples. Such a buttress may prevent the applied staples from pulling through tissue and may otherwise reduce a risk of tissue tearing at or near the site of applied staples.

While various kinds of surgical stapling instruments and associated components have been made and used, it is believed that no one prior to the inventor(s) has made or used the invention described in the appended claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary articulating surgical stapling instrument;
FIG. 2 depicts a perspective view of an end effector of the instrument of FIG. 1, with the end effector in an open configuration;
FIG. 3 depicts an exploded perspective view of the end effector of FIG. 2;
FIG. 4 depicts a perspective view of an exemplary upper buttress and an exemplary lower buttress, each of which may be applied to the end effector of FIG. 2;
FIG. 5A depicts a cross-sectional end view of a portion of the end effector of FIG. 2 with a buttress assembly formed by the buttresses of FIG. 4 applied to the end effector, with tissue positioned between the buttresses in the end effector, and with the anvil in an open position;
FIG. 5B depicts a cross-sectional end view of the combined end effector and buttress assembly of FIG. 5A, with tissue positioned between the buttresses in the end effector, and with the anvil in a closed position;
FIG. 5C depicts a cross-sectional view of a staple and the buttress assembly of FIG. 5A having been secured to the tissue by the end effector of FIG. 2;
FIG. 6 depicts a perspective view of staples and the buttress assembly of FIG. 5A having been secured to the tissue by the end effector of FIG. 2;
FIG. 7 depicts a perspective view of an exemplary alternative buttress;
FIG. 8 depicts a cross-sectional end view of the buttress of FIG. 7;
FIG. 9 depicts a perspective view of another exemplary alternative buttress; and
FIG. 10 depicts a cross-sectional end view of the buttress of FIG. 8.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The invention is defined in the appended claims. The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Surgical Stapler

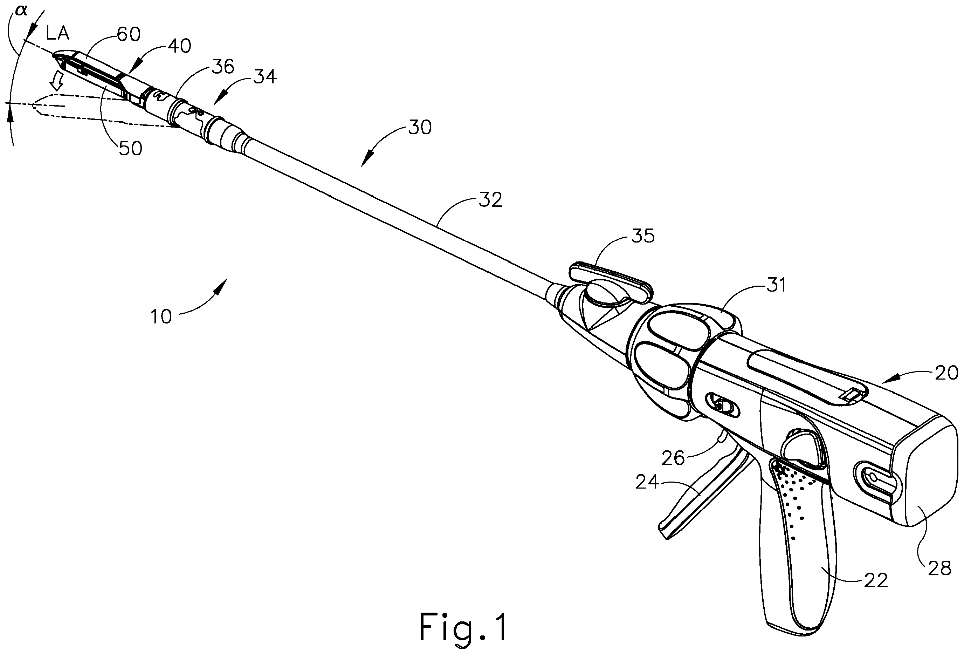

FIG. 1 depicts an exemplary surgical stapling and severing instrument (10) that includes a handle assembly (20), a shaft assembly (30), and an end effector (40). End effector (40) and the distal portion of shaft assembly (30) are sized for insertion, in a nonarticulated state as depicted in FIG. 1, through a trocar cannula to a surgical site in a patient for performing a surgical procedure. By way of example only, such a trocar may be inserted in a patient's abdomen, between two of the patient's ribs, or elsewhere. In some settings, instrument (10) is used without a trocar. For instance, end effector (40) and the distal portion of shaft assembly (30) may be inserted directly through a thoracotomy or other type of incision. It should be understood that terms such as "proximal" and "distal" are used herein with reference to a clinician gripping handle assembly (20) of instrument (10). Thus, end effector (40) is distal with respect to the more proximal handle assembly (20). It will be further appreciated that for convenience and clarity, spatial terms such as "vertical" and "horizontal" are used herein with respect to the drawings. However, surgical instruments are used in many orientations and positions, and these terms are not intended to be limiting and absolute.

### A. Exemplary Handle Assembly and Shaft Assembly

As shown in FIG. 1, handle assembly (20) of the present example comprises pistol grip (22), a closure trigger (24), and a firing trigger (26). Each trigger (24, 26) is selectively pivotable toward and away from pistol grip (22) as will be described in greater detail below. Handle assembly (20) further includes a removable battery pack (28). These components will also be described in greater detail below. Of course, handle assembly (20) may have a variety of other components, features, and operabilities, in addition to or in lieu of any of those noted above. Other suitable configurations for handle assembly (20) will be apparent to those of ordinary skill in the art in view of the teachings herein.

As shown in FIGS. 1-2, shaft assembly (30) of the present example comprises an outer closure tube (32), an articulation section (34), and a closure ring (36), which is further coupled with end effector (40). Closure tube (32) extends along the length of shaft assembly (30). Closure ring (36) is positioned distal to articulation section (34). Closure tube (32) and closure ring (36) are configured to translate longitudinally relative to handle assembly (20). Longitudinal translation of closure tube (32) is communicated to closure ring (36) via articulation section (34). Exemplary features that may be used to provide longitudinal translation of closure tube (32) and closure ring (36) will be described in greater detail below.

Articulation section (34) is operable to laterally deflect closure ring (36) and end effector (40) laterally away from the longitudinal axis (LA) of shaft assembly (30) at a desired angle (α). In the present example, articulation is controlled through an articulation control knob (35) which is located at the proximal end of shaft assembly (30). Closure ring (36) and end effector (40) pivot about an axis that is perpendicular to the longitudinal axis (LA) of shaft assembly (30) in response to rotation of knob (35). Articulation section (34) is configured to communicate longitudinal translation of closure tube (32) to closure ring (36), regardless of whether articulation section (34) is in a straight configuration or an articulated configuration. By way of example only, articulation section (34) and/or articulation control knob (35) may be constructed and operable in accordance with at least some of the teachings of U.S. Pub. No. 2014/0243801, entitled "Surgical Instrument End Effector Articulation Drive with Pinion and Opposing Racks," published August 28, 2014, the disclosure of which is referenced herein; and/or U.S. Pat. App. No. 14/314,125, entitled "Articulation Drive Features for Surgical Stapler," filed June 25, 2014, the disclosure of which is referenced herein; and/or in accordance with the various teachings below. Other suitable forms that articulation section (34) and articulation knob (35) may take will be apparent to those of ordinary skill in the art in view of the teachings herein.

As shown in FIG. 1, shaft assembly (30) of the present example further includes a rotation knob (31). Rotation knob (31) is operable to rotate the entire shaft assembly (30) and end effector (40) relative to handle assembly (20) about the longitudinal axis (LA) of shaft assembly (30). Of course, shaft assembly (30) may have a variety of other components, features, and operabilities, in addition to or in lieu of any of those noted above. By way of example only, at least part of shaft assembly (30) is constructed in accordance with at least some of the teachings of U.S. Pub. No. 2014/0239038, entitled "Surgical Instrument with Multi-Diameter Shaft," published August 28, 2014, the disclosure of which is referenced herein. Other suitable configurations for shaft assembly (30) will be apparent to those of ordinary skill in the art in view of the teachings herein.

### B. Exemplary End Effector

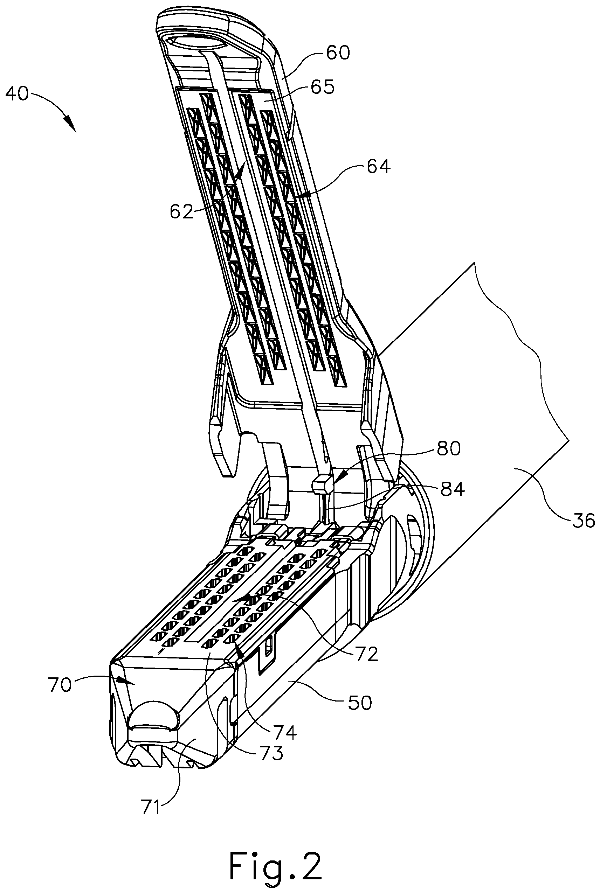

As also shown in FIGS. 1-3, end effector (40) of the present example includes a lower jaw (50) and a pivotable anvil (60). Anvil (60) includes a pair of integral, outwardly extending pins (66) that are disposed in corresponding curved slots (54) of lower jaw (50). Anvil (60) is pivotable toward and away from lower jaw (50) between an open position (shown in FIG. 2) and a closed position (shown in FIG. 1). Use of the term "pivotable" (and similar terms with "pivot" as a base) should not be read as necessarily requiring pivotal movement about a fixed axis. For instance, in the present example, anvil (60) pivots about an axis that is defined by pins (66), which slide along curved slots (54) of lower jaw (50) as anvil (60) moves toward lower jaw (50). In such versions, the pivot axis translates along the path defined by slots (54) while anvil (60) simultaneously pivots about that axis. In addition or in the alternative, the pivot axis may slide along slots (54) first, with anvil (60) then pivoting about the pivot axis after the pivot axis has slid a certain distance along the slots (54). It should be understood that such sliding/translating pivotal movement is encompassed within terms such as "pivot," "pivots," "pivotal," "pivotable," "pivoting," and the like. Of course, some versions may provide pivotal movement of anvil (60) about an axis that remains fixed and does not translate within a slot or channel, etc.

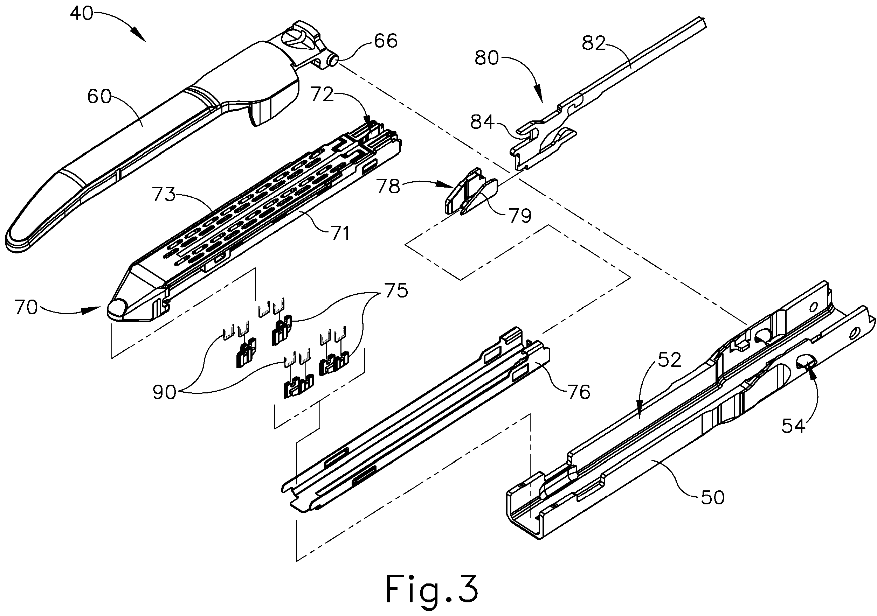

As best seen in FIG. 3, lower jaw (50) of the present example defines a channel (52) that is configured to receive a staple cartridge (70). Staple cartridge (70) may be inserted into channel (52), end effector (40) may be actuated, and then staple cartridge (70) may be removed and replaced with another staple cartridge (70). Lower jaw (50) thus releasably retains staple cartridge (70) in alignment with anvil (60) for actuation of end effector (40). In some versions, lower jaw (50) is constructed in accordance with at least some of the teachings of U.S. Pub. No. 2014/0239044, entitled "Installation Features for Surgical Instrument End Effector Cartridge," published August 28, 2014, the disclosure of which is referenced herein. Other suitable forms that lower jaw (50) may take will be apparent to those of ordinary skill in the art in view of the teachings herein.

As best seen in FIGS. 2-3, staple cartridge (70) of the present example comprises a cartridge body (71) and a tray (76) secured to the underside of cartridge body (71). The upper side of cartridge body (71) presents a deck (73), against which tissue may be compressed when anvil (60) is in a closed position. Cartridge body (71) further defines a longitudinally extending channel (72) and a plurality of staple pockets (74). A staple (90) is positioned in each staple pocket (74). A staple driver (75) is also positioned in each staple pocket (74), underneath a corresponding staple (90), and above tray (76). As will be described in greater detail below, staple drivers (75) are operable to translate upwardly in staple pockets (74) to thereby drive staples (90) upwardly through staple pockets (74) and into engagement with anvil (60). Staple drivers (75) are driven upwardly by a wedge sled (78), which is captured between cartridge body (71) and tray (76), and which translates longitudinally through cartridge body (71).

Wedge sled (78) includes a pair of obliquely angled cam surfaces (79), which are configured to engage staple drivers (75) and thereby drive staple drivers (75) upwardly as wedge sled (78) translates longitudinally through cartridge (70). For instance, when wedge sled (78) is in a proximal position, staple drivers (75) are in downward positions and staples (90) are located in staple pockets (74). As wedge sled (78) is driven to the distal position by a translating knife member (80), wedge sled (78) drives staple drivers (75) upwardly, thereby driving staples (90) out of staple pockets (74) and into staple forming pockets (64) that are formed in the underside (65) of anvil (60). Thus, staple drivers (75) translate along a vertical dimension as wedge sled (78) translates along a horizontal dimension.

In some versions, staple cartridge (70) is constructed and operable in accordance with at least some of the teachings of U. U.S. Pub. No. 2014/0239042, entitled "Integrated Tissue Positioning and Jaw Alignment Features for Surgical Stapler," published August 28, 2014, the disclosure of which is referenced herein. In addition or in the alternative, staple cartridge (70) may be constructed and operable in accordance with at least some of the teachings of U.S. Pub. No. 2014/0239044, entitled "Installation Features for Surgical Instrument End Effector Cartridge," published August 28, 2014, the disclosure of which is referenced herein. Other suitable forms that staple cartridge (70) may take will be apparent to those of ordinary skill in the art in view of the teachings herein.

As best seen in FIG. 2, anvil (60) of the present example comprises a longitudinally extending channel (62) and a plurality of staple forming pockets (64). Channel (62) is configured to align with channel (72) of staple cartridge (70) when anvil (60) is in a closed position. Each staple forming pocket (64) is positioned to lie over a corresponding staple pocket (74) of staple cartridge (70) when anvil (60) is in a closed position. Staple forming pockets (64) are configured to deform the legs of staples (90) when staples (90) are driven through tissue and into anvil (60). In particular, staple forming pockets (64) are configured to bend the legs of staples (90) to secure the formed staples (90) in the tissue. Anvil (60) may be constructed in accordance with at least some of the teachings of U.S. Pub. No. 2014/0239042, entitled "Integrated Tissue Positioning and Jaw Alignment Features for Surgical Stapler," published August 28, 2014; at least some of the teachings of U.S. Pub. No. 2014/0239036, entitled "Jaw Closure Feature for End Effector of Surgical Instrument," published August 28, 2014; and/or at least some of the teachings of U.S. Pub. No. 2014/0239037, entitled "Staple Forming Features for Surgical Stapling Instrument," published August 28, 2014, the disclosure of which is referenced herein. Other suitable forms that anvil (60) may take will be apparent to those of ordinary skill in the art in view of the teachings herein.

In the present example, a knife member (80) is configured to translate through end effector (40). As best seen in FIG. 3, knife member (80) is secured to the distal end of a firing beam (82), which extends through a portion of shaft assembly (30). As best seen in FIG. 2, knife member (80) is positioned in channels (62, 72) of anvil (60) and staple cartridge (70). Knife member (80) includes a distally presented cutting edge (84) that is configured to sever tissue that is compressed between anvil (60) and deck (73) of staple cartridge (70) as knife member (80) translates distally through end effector (40). As noted above, knife member (80) also drives wedge sled (78) distally as knife member (80) translates distally through end effector (40), thereby driving staples (90) through tissue and against anvil (60) into formation.

### C. Exemplary Actuation of End Effector

In the present example, anvil (60) is driven toward lower jaw (50) by advancing closure ring (36) distally relative to end effector (40). Closure ring (36) cooperates with anvil (60) through a camming action to drive anvil (60) toward lower jaw (50) in response to distal translation of closure ring (36) relative to end effector (40). Similarly, closure ring (36) may cooperate with anvil (60) to open anvil (60) away from lower jaw (50) in response to proximal translation of closure ring (36) relative to end effector (40). By way of example only, closure ring (36) and anvil (60) may interact in accordance with at least some of the teachings of U.S. Pub. No. 2014/0239036, entitled "Jaw Closure Feature for End Effector of Surgical Instrument," published August 28, 2014, the disclosure of which is referenced herein; and/or in accordance with at least some of the teachings of U.S. Patent App. No. 14/314,108, entitled "Jaw Opening Feature for Surgical Stapler," filed on June 25, 2014, the disclosure of which is referenced herein.

As noted above, handle assembly (20) includes a pistol grip (22) and a closure trigger (24). As also noted above, anvil (60) is closed toward lower jaw (50) in response to distal advancement of closure ring (36). In the present example, closure trigger (24) is pivotable toward pistol grip (22) to drive closure tube (32) and closure ring (36) distally. Various suitable components that may be used to convert pivotal movement of closure trigger (24) toward pistol grip (22) into distal translation of closure tube (32) and closure ring (36) relative to handle assembly (20) will be apparent to those of ordinary skill in the art in view of the teachings herein.

Also in the present example, instrument (10) provides motorized control of firing beam (82). In particular, instrument (10) includes motorized components that are configured to drive firing beam (82) distally in response to pivoting of firing trigger (26) toward pistol grip (22). In some versions, a motor (not shown) is contained in pistol grip (22) and receives power from battery pack (28). This motor is coupled with a transmission assembly (not shown) that converts rotary motion of a drive shaft of the motor into linear translation of firing beam (82). By way of example only, the features that are operable to provide motorized actuation of firing beam (82) may be configured and operable in accordance with at least some of the teachings of U.S. Pat. No. 8,210,411, entitled "Motor-Driven Surgical Instrument," issued July 3, 2012, the disclosure of which is referenced herein.; U.S. Pat. No. 8,453,914, entitled "Motor-Driven Surgical Cutting Instrument with Electric Actuator Directional Control Assembly," issued June 4, 2013, the disclosure of which is referenced herein; and/or U.S. Patent App. No. 14/226,142, entitled "Surgical Instrument Comprising a Sensor System," filed March 26, 2014, the disclosure of which is referenced herein.

It should also be understood that any other components or features of instrument (10) may be configured and operable in accordance with any of the various references cited herein. Additional exemplary modifications that may be provided for instrument (10) will be described in greater detail below. Various suitable ways in which the below teachings may be incorporated into instrument (10) will be apparent to those of ordinary skill in the art. Similarly, various suitable ways in which the below teachings may be combined with various teachings of the references cited herein will be apparent to those of ordinary skill in the art. It should therefore be understood that the teachings below may be readily incorporated into the various instruments taught in the various references that are cited herein. It should also be understood that the below teachings are not limited to instrument (10) or devices taught in the references cited herein. The below teachings may be readily applied to various other kinds of instruments, including instruments that would not be classified as surgical staplers. Various other suitable devices and settings in which the below teachings may be applied will be apparent to those of ordinary skill in the art in view of the teachings herein.

### II. Exemplary Buttress Assembly for Surgical Stapler

In some instances, it may be desirable to equip end effector (40) with a buttress material to reinforce the mechanical fastening of tissue provided by staples (90). Such a buttress may prevent the applied staples (90) from pulling through the tissue and may otherwise reduce a risk of tissue tearing at or near the site of applied staples (90). In addition to or as an alternative to providing structural support and integrity to a line of staples (90), a buttress may provide various other kinds of effects such as spacing or gap-filling, administration of therapeutic agents, and/or other effects. In some instances, a buttress may be provided on deck (73) of staple cartridge (70). In some other instances, a buttress may be provided on the surface of anvil (60) that faces staple cartridge (70). It should also be understood that a first buttress may be provided on deck (73) of staple cartridge (70) while a second buttress is provided on anvil (60) of the same end effector (40). Various examples of forms that a buttress may take will be described in greater detail below. Various ways in which a buttress may be secured to a staple cartridge (70) or an anvil (60) will also be described in greater detail below.

### A. Exemplary Composition of Buttress Assembly for Surgical Stapler

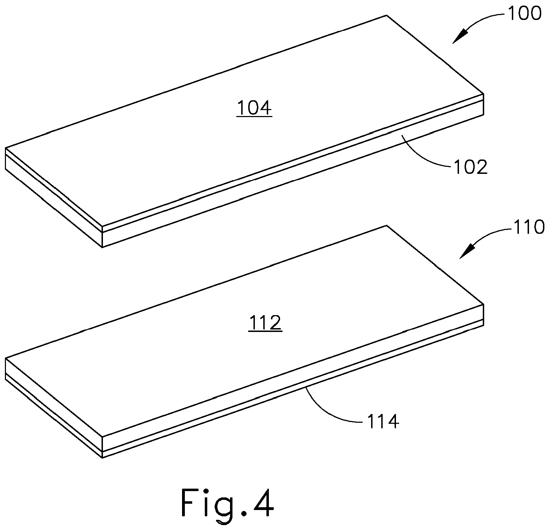

FIG. 4 shows an exemplary pair of buttress assemblies (100, 110) with a basic composition. Buttress assembly (100) of this example comprises a buttress body (102) and an upper adhesive layer (104). Similarly, buttress assembly (110) comprises a buttress body (112) and a lower adhesive layer (114). In the present example, each buttress body (102, 112) comprises a strong yet flexible material configured to structurally support a line of staples (90). By way of example only, each buttress body (102, 112) may comprise a woven mesh of polyglactin 910 material by Ethicon, Inc. of Somerville, New Jersey. Alternatively, any other suitable materials or combinations of materials may be used in addition to or as an alternative to polyglactin 910 material to form each buttress body (102, 112). Each buttress body (102, 112) may take any other suitable form and may be constructed of any other suitable material(s). By way of further example only, each buttress body (102, 112) may comprise one or more of the following: NEOVEIL absorbable PGA felt by Gunze Limited, of Kyoto, Japan; SEAMGUARD polyglycolic acid:trimethylene carbonate (PGA:TMC) reinforcement material by W.L. Gore & Associates, Inc., of Flagstaff, Arizona; PERI-STRIPS DRY with VERITAS Collagen Matrix (PSDV) reinforcement material, by Baxter Healthcare Corporation of Deerfield, Illinois; BIODESIGN biologic graft material by Cook Medical, Bloomington, Indiana; and/or SURGICEL NU-KNIT hemostat material by Ethicon, Inc. of Somerville, New Jersey. Still other suitable materials that may be used to form each buttress body (102, 112) will be apparent to those of ordinary skill in the art in view of the teachings herein.

In addition or in the alternative, each buttress body (102, 112) may comprise a material including, for example, a hemostatic agent such as fibrin to assist in coagulating blood and reduce bleeding at the severed and/or stapled surgical site along tissue (90). As another merely illustrative example, each buttress body (102, 112) may comprise other adjuncts or hemostatic agents such as thrombin may be used such that each buttress body (102, 112) may assist to coagulate blood and reduce the amount of bleeding at the surgical site. Other adjuncts or reagents that may be incorporated into each buttress body (102, 112) may further include but are not limited to medical fluid or matrix components. Merely illustrative examples of materials that may be used to form each buttress body (102, 112), as well as materials that may be otherwise incorporated into each buttress body (102, 112), are disclosed in U.S. Patent App. No. 14/667,842, entitled "Method of Applying a Buttress to a Surgical Stapler," filed March 25, 2015, the disclosure of which is referenced herein. Alternatively, any other suitable materials may be used.

By way of further example only, each buttress body (102, 112) may be constructed in accordance with at least some of the teachings of U.S. Patent Pub. No. 2012/0241493, entitled "Tissue Thickness Compensator Comprising Controlled Release and Expansion," published September 27, 2012, the disclosure of which is referenced herein; U.S. Patent Pub. No. 2013/0068816, entitled "Surgical Instrument and Buttress Material," published March 21, 2013, the disclosure of which is referenced herein; U.S. Patent Pub. No. 2013/0062391, entitled "Surgical Instrument with Fluid Fillable Buttress," published March 14, 2013, the disclosure of which is referenced herein; U.S. Patent Pub. No. 2013/0068820, entitled "Fibrin Pad Matrix with Suspended Heat Activated Beads of Adhesive," published March 21, 2013, the disclosure of which is referenced herein; U.S. Patent Pub. No. 2013/0082086, entitled "Attachment of Surgical Staple Buttress to Cartridge," published April 4, 2013, the disclosure of which is referenced herein; U.S. Patent Pub. No. 2013/0037596, entitled "Device for Applying Adjunct in Endoscopic Procedure," published February 14, 2013, the disclosure of which is referenced herein; U.S. Patent Pub. No. 2013/0062393, entitled "Resistive Heated Surgical Staple Cartridge with Phase Change Sealant," published March 14, 2013, the disclosure of which is referenced herein; U.S. Patent Pub. No. 2013/0075446, entitled "Surgical Staple Assembly with Hemostatic Feature," published March 28, 2013, the disclosure of which is referenced herein; U.S. Patent Pub. No. 2013/0062394, entitled "Surgical Staple Cartridge with Self-Dispensing Staple Buttress," published March 14, 2013, the disclosure of which is referenced herein; U.S. Patent Pub. No. 2013/0075445, entitled "Anvil Cartridge for Surgical Fastening Device," published March 28, 2013, the disclosure of which is referenced herein; U.S. Patent Pub. No. 2013/0075447, entitled "Adjunct Therapy for Applying Hemostatic Agent," published March 28, 2013, the disclosure of which is referenced herein; U.S. Patent Pub. No. 2013/0256367, entitled "Tissue Thickness Compensator Comprising a Plurality of Medicaments," published October 3, 2013, the disclosure of which is referenced herein; U.S. Patent Application No. 14/300,954, entitled "Adjunct Materials and Methods of Using Same in Surgical Methods for Tissue Sealing," filed June 10, 2014, the disclosure of which is referenced herein; U.S. Patent App. No. 14/827,856, entitled "Implantable Layers for a Surgical Instrument," filed August 17, 2015, the disclosure of which is referenced herein; U.S. Patent Application No. 14/840,613, entitled "Drug Eluting Adjuncts and Methods of Using Drug Eluting Adjuncts," filed August 31, 2015, the disclosure of which is referenced herein; U.S. Patent App. No. 14/871,071, entitled "Compressible Adjunct with Crossing Spacer Fibers," filed September 30, 2015, the disclosure of which is referenced herein; and/or U.S. Patent App. No. 14/871,131, entitled "Method for Applying an Implantable Layer to a Fastener Cartridge," filed September 30, 2015, the disclosure of which is referenced herein.

In the present example, adhesive layer (104) is provided on buttress body (102) in order to adhere buttress body (102) to underside (65) of anvil (60). Similarly, adhesive layer (114) is provided on buttress body (112) in order to adhere buttress body (112) to deck (73) of staple cartridge (70). Adherence of the buttress body (102) to underside (65) of anvil (60) or to deck (73) of staple cartridge (70) can occur through a variety of mechanisms including but not limited to a pressure sensitive adhesive. In some versions, each adhesive layer (104, 114) comprise a pressure sensitive adhesive material. Examples of various suitable materials that may be used to form adhesive layers (104, 114) are disclosed in U.S. Patent App. No. 14/667,842, entitled "Method of Applying a Buttress to a Surgical Stapler," filed March 25, 2015, the disclosure of which is referenced herein. Alternatively, any other suitable materials may be used. It should be understood that the term "adhesive," as used herein, may include (but is not limited to) tacky materials and also materials that arc pliable or wax-likc and adhere to a complex geometry via deformation and conformance. Some suitable adhesives may provide such pliability to adhere to a complex geometry via deformation and conformance without necessarily providing a high initial tack. In some instances, adhesives with lower tackiness may be removed more cleanly from surfaces. Various suitable materials that may be used to form adhesive layers (104, 114) will be apparent to those of ordinary skill in the art in view of the teachings herein.

### B. Exemplary Materials and Techniques for Providing Adhesion of Buttress to Surgical Stapler

As noted above, a buttress assembly (100, 110) may include a layer (104, 114) of adhesive material (or other form of adhesive material) that adheres buttress body (102, 112) to either underside (65) of anvil (60) or deck (73) of staple cartridge (70). Such an adhesive material may provide proper positioning of buttress body (102, 112) before and during actuation of end effector (40); then allow buttress body (102, 112) to separate from end effector (40) after end effector (40) has been actuated, without causing damage to buttress body (102, 112) that is substantial enough to compromise the proper subsequent functioning of buttress body (102, 112).

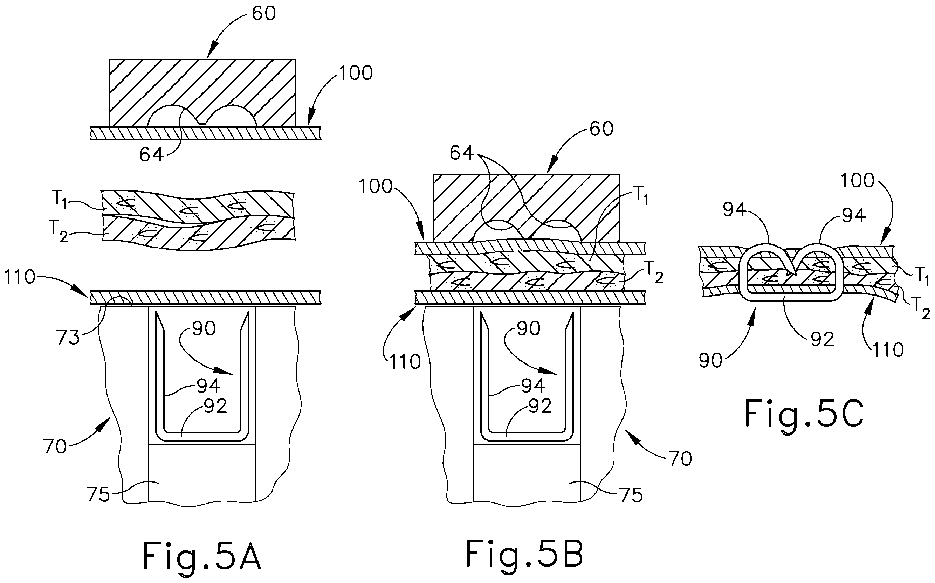

FIGS. 5A-5C show a sequence where an end effector (40) that has been loaded with buttress assemblies (100, 110) is actuated to drive staples (90) through two apposed layers of tissue (T₁, T₂), with buttress assemblies (100, 110) being secured to the same layers of tissue (T₁, T₂) by staples (90). In particular, FIG. 5A shows layers of tissue (T₁, T₂) positioned between anvil (60) and staple cartridge (70), with anvil (60) in the open position. Buttress assembly (100) is adhered to the underside (65) of anvil (60) via adhesive layer (104); while buttress assembly (110) is adhered to deck (73) of staple cartridge (70) via adhesive layer (114). Layers of tissue (T₁, T₂) are thus interposed between buttress assemblies (100, 110). Next, trigger (24) is pivoted toward pistol grip (22) to drive closure tube (32) and closure ring (36) distally. This drives anvil (60) to the closed position as shown in FIG. 5B. At this stage, layers of tissue (T₁, T₂) are compressed between anvil (60) and staple cartridge (70), with buttress assemblies (100, 110) engaging opposite surfaces of tissue layers (T₁, T₂). End effector (40) is then actuated as described above, driving staple (90) through buttress assemblies (100, 110) and tissue (90). As shown in FIG. 5C, crown (92) of driven staple (90) captures and retains buttress assembly (110) against layer of tissue (T₂). Deformed legs (94) of staple (90) capture and retain buttress assembly (100) against layer of tissue (T₁).

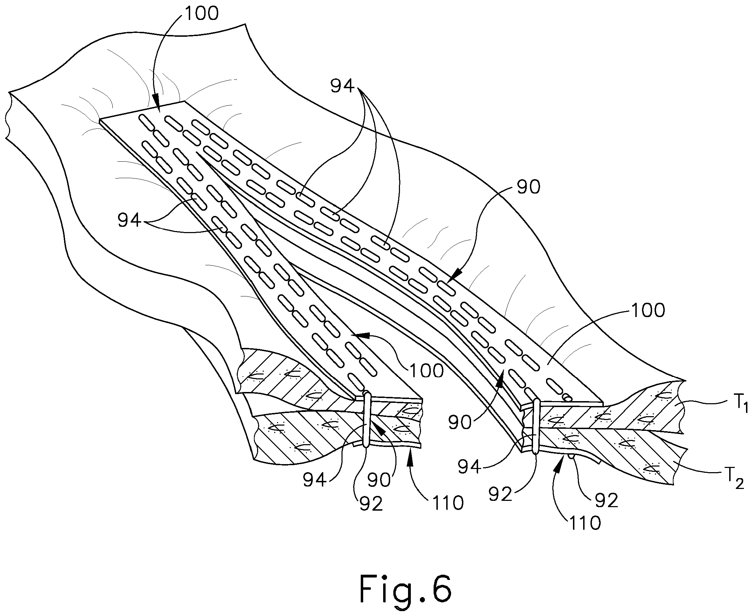

It should be understood that a series of staples (90) will similarly capture and retain buttress assemblies (100, 110) against layers of tissue (T₁, T₂), thereby securing buttress assemblies (100, 110) to tissue (T₁, T₂) as shown in FIG. 6. As end effector (40) is pulled away from tissue (90) after deploying staples (90) and buttress assemblies (100, 110), buttress assemblies (100, 110) disengage end effector), such that buttress assemblies (100, 110) remain secured to tissue (T₁, T₂) with staples (90). Buttress tissue (T₁, T₂) thus provide structural reinforcement to the lines of staples (90). As can also be seen in FIG. 6, knife member (80) also cuts through a centerline of buttress tissue assemblies (100, 110), separating each buttress assemblies (100, 110) into a corresponding pair of sections, such that each section remains secured to a respective severed region of tissue (T₁, T₂).

In the foregoing example, buttress assembly (100) is sized to span across the full width of underside (65), such that buttress assembly (100) spans across channel (62). Thus, knife member (80) cuts through buttress assembly (100) during actuation of end effector (40) as described above. In some other examples, such as those described below, buttress assembly (100) is provided in two separate, laterally spaced apart portions, with one portion being disposed on underside (65) on one side of channel (62) and another portion being disposed on underside (65) on the other side of channel (62). In such versions, buttress assembly (100) does not span across channel (62), such that knife member (80) does not cut through buttress assembly (100) during actuation of end effector (40).

Likewise, buttress assembly (110) may be sized to span across the full width of deck (73), such that buttress assembly (110) spans across channel (72), and such that knife member (80) cuts through buttress assembly (110) during actuation of end effector (40) as described above. Alternatively, buttress assembly (110) may be provided in two separate, laterally spaced apart portions, with one portion being disposed on deck (73) on one side of channel (72) and another portion being disposed on deck (73) on the other side of channel (72), such that buttress assembly (110) does not span across channel (72), and such that knife member (80) does not cut through buttress assembly (110) during actuation of end effector (40).

### III. Exemplary Multi-Layer Adhesive Arrangement for Buttress Assembly

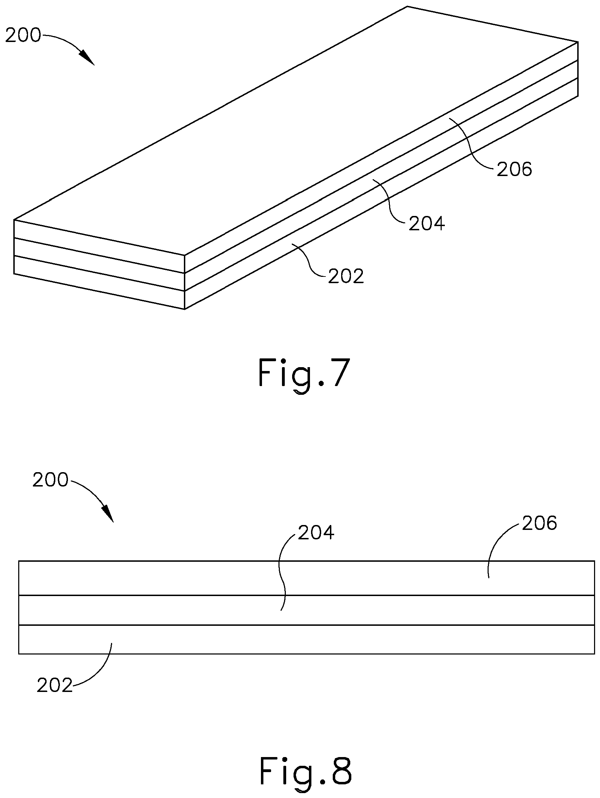

In some instances, it may be desirable to provide a version of a buttress assembly (100, 110) where adhesive layer (104, 114) comprises two or more layers of different kinds of adhesive material having different properties. For instance, FIGS. 7-8 show an exemplary buttress assembly (200) that comprises a buttress body (202), a first adhesive layer (204) laid over buttress body (202), and a second adhesive layer (206) laid over first adhesive layer (204). It should be understood that, with buttress body (202) at the bottom of buttress assembly (200), buttress assembly (200) is analogous to buttress assembly (100) described above and may be similarly secured to underside (65) of anvil (60). Alternatively, buttress assembly (200) may be flipped upside-down for an arrangement where buttress assembly (200) would be secured to deck (73) of staple cartridge (70). In such versions, second adhesive layer (206) would be at the bottom, first adhesive layer (204) would be laid over second adhesive layer (206), and buttress body (202) would be laid over first adhesive layer (204).

Buttress body (202) may be constructed and operable just like buttress bodies (102, 112) described above. Moreover, buttress body (202) may be constructed and operable in accordance with at least some of the teachings of U.S. Patent App. No. 14/667,842, entitled "Method of Applying a Buttress to a Surgical Stapler," filed March 25, 2015, the disclosure of which is referenced herein. Other suitable forms that buttress body (202) may take will be apparent to those of ordinary skill in the art in view of the teachings herein.

First adhesive layer (204) is formed of an adhesive material that is different from the adhesive material forming second adhesive layer (206). In some versions, first adhesive layer (204) is formed of a material that has greater pliability and tackiness than the material forming second adhesive layer (206). In some such versions, second adhesive layer (206) serves as a protectant for first adhesive layer (204). For instance, second adhesive layer (206) may protect first adhesive layer (204) from humidity, temperature fluctuations, and/or other environmental conditions that may be encountered during shipment and/or storage of buttress assembly (200). In other words, second adhesive layer (206) may be more resistant to moisture and/or temperature than first adhesive layer (206). In some other variations, second adhesive layer (206) is replaced with a non-adhesive protective layer. By way of example only, second adhesive layer (206) may be replaced by a film or other structure that is biocompatible and bioabsorbable, dissolvable, or otherwise capable of temporarily confining the material forming first adhesive layer (204) (e.g., to prevent first adhesive layer from seeping, migrating, or otherwise flowing out of buttress assembly (200) during storage, shipping, handling before surgery, etc.). When anvil (60) is pressed against buttress assembly (200) both adhesive layers (204, 206) may nevertheless cooperate to adhere buttress assembly (200) to underside (65) of anvil (60).

In some versions, second adhesive layer (206) is sprayed onto first adhesive layer (204) while first adhesive layer is maintained at a temperature and humidity level that keeps first adhesive layer (204) solid. It should be understood that the combination of different adhesive layers (204, 206) may be more soluble against anvil (60) and/or less sticky against anvil (60) than just a single adhesive layer (204) might be. This may prevent an undesirable buildup of adhesive material on underside (65) of anvil (60) as a series of buttress assemblies (200) are applied to underside (65) for a series of end effector (40) actuations during a surgical procedure. It should also be understood that adhesive layers (204, 206) may provide different ratios of two molecular weight blends in different layers (204, 206). Unless otherwise stated, the term molecular weight means weight average molecular weight herein. In addition, adhesive layers (204, 206) may have cross-linking differences and/or come from different families of adhesive. By way of further example only, adhesive layers (204, 206) formed by higher molecular weight poloxamers may be stiffer and less prone to flow with temperature. Thus, using a poloxamer blend with higher molecular weight to form adhesive layer (206) may contain a lower molecular weight material forming adhesive layer (204), even if adhesive layer (204) becomes fluid with temperature. Materials with higher degrees of cross-linking may have higher transition temperatures and therefore flow less at temperature extremes. An example of different families could be a poloxamer blend in adhesive layer (204) with a thin layer of PCL/PGA co-ploymer sprayed onto adhesive layer (204) to form adhesive layer (206).

Various suitable materials that may be used to form second adhesive layer (206) and non-adhesive substitutes for second adhesive layer will be apparent to those of ordinary skill in the art in view of the teachings herein. By way of example only, first adhesive layer (204) may be configured and operable in accordance with at least some of the teachings of U.S. Pat. App. No. 14/926,045, entitled "Surgical Stapler Buttress Assembly with Humidity Tolerant Adhesive," filed on even date herewith, the disclosure of which is referenced herein; U.S. Pat. App. No. 14/926,057, entitled "Surgical Stapler Buttress Assembly with Adhesion to Wet End Effector," filed on even date herewith, the disclosure of which is referenced herein; and/or any other references cited herein.

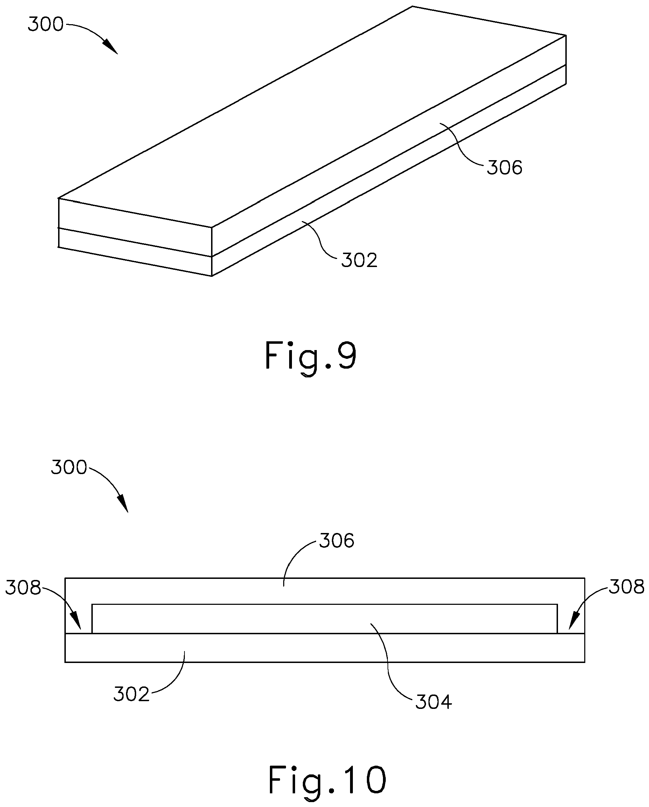

In buttress assembly (200), the outer edges of first adhesive layer (204) are left exposed by second adhesive layer (206). FIGS. 9-10 show an exemplary alternative buttress assembly (300) where the outer edges of a first adhesive layer (304) are covered by downwardly projecting regions (308) of a second adhesive layer (306). Downwardly projecting regions (308) extend along the full width and the full length of firs adhesive layer (304) in this example. Downwardly projecting regions (308) also extend downwardly into contact with the upper surface of buttress body (302). Buttress body (302) and second adhesive layer (306) thus cooperate to completely encapsulate first adhesive layer (304). Buttress body (302), first adhesive layer (304), and second adhesive layer (306) may be otherwise identical to buttress body (202), first adhesive layer (204), and second adhesive layer (206) as described above. Other suitable ways in which adhesive layers (304, 306) may be configured and arranged in relation to a buttress body (302) will be apparent to those of ordinary skill in the art in view of the teachings herein.

In addition to the foregoing, it should also be understood that any of the various buttress assemblies described herein may be further constructed and operable in accordance with at least some of the teachings of U.S. Patent App. No. 14/667,842, entitled "Method of Applying a Buttress to a Surgical Stapler," filed March 25, 2015, the disclosure of which is referenced herein.

### IV. U.S. Pat. App. No. 14/926,045 describes:

In some instances, the humidity tolerant adhesive materials (e.g., one or more of layers (104, 204, 304, 114)) for a buttress body (102, 202, 302, 112) comprise bioabsorbable polymers. Various physiomechanical properties of polymers may be modified in order to provide different adhesive properties. Such variable characteristics include but are not limited to copolymer composition, polymer architecture (e.g., random vs. block copolymers and/or branching), glass transition temperature (Tg), molecular weight (number average or weight average molecular weight), inherent viscosity (IV), crystallinity, sequence distribution, copolymer chain composition, melting temperature (Tm), surface tension and rheological properties. Several exemplary combinations of these variables will be provided below, though it should be understood that these examples are merely illustrative. It should also be understood that these examples of adhesive materials may be provided in upper adhesive layer (104, 204, 304). In addition or in the alternative, these examples of adhesive materials may be provided in lower adhesive layer (114). In addition or in the alternative, these examples of adhesive materials may be otherwise integrated into buttress body (102, 202, 302, 112). It should therefore be understood that the adhesive material need not necessarily constitute a separate layer that is discretely identifiable as being different from a layer defined by buttress body (102, 202, 302, 112).

One of the aforementioned physiomechanical properties of polymers is glass transition temperature (Tg). Glass transition temperature (Tg) is the temperature at which the mechanical properties of a copolymer change dramatically from a flowable adhesive to a brittle plastic. It may thus be of importance that the glass transition temperature (Tg) is sufficiently below the operating temperature of the adhesive in order to allow for polymer chain mobility. The glass transition temperature (Tg) is lower than the melting point of the crystalline form of the same copolymer. The glass transition temperature (Tg) may be indicative of how the polymer behaves under ambient conditions. The glass transition temperature (Tg) can be effected by composition, polymer chain configuration and stiffness, molecular weight, viscosity, shear modulus, heat capacity, thermal expansion, cross-linking and other factors. It is therefore possible to have a relatively low glass transition temperature (Tg) material composition that does not always correspond to low molecular weight or low inherent viscosity (IV).

The melting temperature of a polymer may be referred to as the "first-order transition," which is where the polymer changes from a solid to liquid. Crystalline polymers have a true melting point, which is the temperature at which the crystallites melt and the total mass of plastic becomes amorphous. Amorphous polymers do not have a true melting point, but they do have a first-order transition wherein their mechanical behavior transitions from a rubbery nature to viscous rubbery flow. Suitable polymers for use in forming adhesive layers (104, 204, 304, 114) may have a percentage of crystallinity making them semi-crystalline, thus having both amorphous and crystalline domains. The melting point of the polymer may be sufficiently high above the operating temperature of the adhesive to maintain cohesive strength and provide dimensional stability of the applied adhesive.

Inherent viscosity (IV) reflects a measurement of molecular size. It is based on the flow time of a polymer solution through small capillary channels over time. The inherent viscosity (IV) and molecular weight of a polymer are related, but that relational agreement is different for each copolymer composition. For instance, the correlation of inherent viscosity (IV) to molecular weight may be logarithmic with only a small midsection of the curve being linear. This logarithmic correlation may differ as the copolymer composition differs. It is not necessarily required to have a low molecular weight copolymer in order to manifest adhesive and malleable properties. Low molecular weight copolymers may also have shortened degradation cycles and reduced structural strength. The ideal adhesion film or adhesive substrate to use in adhesive layers (104, 204, 304, 114) may have higher molecular weight and low inherent viscosity (IV) to be both strong and adhesive. This may be achieved, for example, by the introduction of polymer branching. The molecular weight of the adhesive may need to be high enough to provide mechanical strength to the adhesive to avoid cohesive failure, but also sufficiently low enough that it can be cleared from the body through degradation in an acceptable amount of time.

Further important properties of the polymers include their surface tension and rheological properties. If there is a sufficiently large mismatch between the surface tension of the adhesive and the surfaces to be adhered to, adhesion may be energetically unfavorable. Similarly, the rheological properties of the polymer such as bulk moduli may need to be such that the polymer can flow to conform to the surface topography of deck (73) or underside (65), while simultaneously providing enough integrity to maintain cohesive strength and to resist shearing off and/or peeling off of end effector (40).

In some instances, the humidity tolerant adhesive materials may be malleable. Malleable humidity tolerant adhesives may be highly viscous yet flowable at room temperature. A malleable humidity tolerant adhesive may, in response to pressure being applied to it, take the form of a surface with which it is engaged. In other words, if a malleable humidity tolerant adhesive is pressed against deck (73) of staple cartridge (70), the adhesive may take the form of the one or more features of the deck (73) that it the adhesive is pressed against. Similarly, if a malleable humidity tolerant polymer adhesive is pressed against underside (65) of anvil (60), the adhesive may take the form of the one or more features of underside (65) that the adhesive is pressed against. By deforming to the geometry that it is pressed against, the malleable humidity tolerant adhesive may adhere to the geometry, and may further provide re-applicable attachment. If the desired positioning of buttress assembly (100, 110) on deck (73) or underside (65) is not achieved, the malleable humidity tolerant adhesive may permit buttress assembly (100, 110) to be removed, repositioned, and re-adhered to deck (73) or underside (65). It should be understood that the humidity tolerant adhesives may be malleable at room temperature, such that additional heating or other treatment is not necessary in order to provide malleability.

Providing the humidity tolerant adhesive material in the form of a malleable polymer may minimize the impact of fluids and debris on the adhesion of buttress assembly (100, 110) to deck (73) of staple cartridge (70) or underside (65) of anvil (60). The malleable humidity tolerant adhesive material may also be hydrophilic (e.g., at least in certain regions of buttress assembly (100, 110)), encouraging adhesion in a wet environment. In addition or in the alternative, adhesive layer (104, 114) of buttress assembly (100) may include a combination of adhesive material and hydrophobic material in respective localized regions. The hydrophobic material may drive fluids out of the adhesion areas, thereby improving adhesion at the localized regions of adhesive material. In some examples, the humidity tolerant adhesive material may be combined with a buttress body (102,112) as disclosed in U.S. Patent App. No. 14/667,842, entitled "Method of Applying a Buttress to a Surgical Stapler," filed March 25, 2015, the disclosure of which is referenced herein.

In some instances, the humidity tolerant adhesive materials may be extrudable. The extrudable adhesive may be extruded through a die that may be positioned directly next to or adjacent to the extruder. A melt pump may be used between the die and extruder. The die may be used to form an extrudate that is generally planar and continuous or to form discrete deposits (e.g., rod-shaped deposits) on the surface of a buttress assembly (100, 110) before it is pressed against a deck (73) of staple cartridge (70) or pressed against the underside of an anvil (60).

### A. Exemplary Humidity Tolerant Adhesives with A-B-A Block Polymer Configurations

In some instances, the humidity tolerant adhesive materials (e.g., one or more of layers (104, 204, 304, 114)) for a buttress body (102, 202, 302, 112) comprise polymers having a general A-B-A block configuration. In illustrative examples, the A-B-A block polymers comprise by the percentage of their molecular weight: from about 1% to about 50%, or more particularly from about 5% to about 30% of A polymer blocks; and from about 50% to about 99%, or more particularly from about 70% to about 95%, of B polymer blocks.

The A polymer blocks are biodegradable, bioabsorbable, highly crystalline segments, which are homopolymers that may be characterized by a relatively high glass transition temperature (Tg) and/or a relatively high crystallinity. In illustrative examples, the A homopolymers may be characterized by a glass transition temperature (Tg) of at least about 0°C, preferably at least about 21°C (i.e., room temperature). In addition, or in the alternative, the A homopolymers may be characterized by a crystallinity as measured by X-ray diffraction of at least about 30%, preferably at least about 40%, or more preferably at least about 45%. In addition, or in the alternative, the A homopolymers may have a molecular weight of at least about 5 kDa. Such exemplary A homopolymers may further be characterized by a melting temperature (Tm) of at least about 50°C, preferably at least about 60°C, and more preferably at least about 70°C.

Exemplary A homopolymers may be selected from the group of: poly(L-lactide) (PLLA); poly(caprolactone) (PCL); polyglycolide (PGA); poly(3-hydroxybutyrate) (PH₃B); poly(3-hydroxyvalerate) (PHV); and poly(p-dioxanone) (PPDO). It may be difficult to synthesize 100% A homopolymers. In some instances, the A homopolymers may contain a small percentage of residual B monomers. For example, exemplary A homopolymers may contain a small percentage (e.g., up to about 10% by weight) of B monomers.

The B polymer blocks are biodegradable, bioabsorbable homopolymers or co-polymers, which are predominantly amorphous and may be characterized by a relatively low to moderate glass transition temperature (Tg). In illustrative examples, the B polymers as homopolymers or co-polymers may be characterized by glass transition temperature (Tg) of at least about -40°C, more particularly at least about -30°C, and more particularly at least about -20 °C. In addition, or in the alternative, the B homopolymers or co-polymers may be characterized by a crystallinity as measured by X-ray diffraction of at most about 25%, more particularly at most about 10%, or more particularly at most about 5%. In addition, or in the alternative, the B polymers as homopolymers or co-polymers may have a molecular weight of from about 20 to about 80 kDa, more particularly from about 30 to about 70 kDa, and more particularly, from about 40 to about 65 kDa.

Exemplary B homopolymers or co-polymers comprise monomers selected from the group consisting of: caprolactone (CL), L-Lactide (LLA), D,L-Lactide ((D,L)LA), Glycolide (GA), Polydioxanone (PDO), Trimethylene carbonate (TMC), sebacic acid (SA), 1,6-bis(carboxyphenoxy)hexane (CPH), and combinations thereof.

In some instances, the humidity tolerant adhesive materials having a general A-B-A block configuration may be blended with a tackifying agent to provide for an extrudable adhesive. Such extrudable humidity tolerant adhesive materials may be manufactured using hot melt extrusion. In illustrative examples, the A-B-A block polymer may be fed into a hot melt compounding twin-screw extruder. Once the A-B-A block polymer is sufficiently masticated and melted, the tackifying agent is added into the extruder. In some versions, additional compounds may be added in one or more additional steps to the extruder. Such additional compounds may selected from the group consisting of: plasticizing molecules; preservatives (e.g. antioxidants); fillers; and combinations thereof. Once mixing in the extruder is completed, the resulting adhesive may be fed through an extruder die and produced as a stand-alone flexible film that is then applied to a buttress body (102, 112). In addition, or in the alternative, the resulting adhesive may be fed through an extruder die and deposited directly onto a buttress body (102, 112). In any case, the adhesive may then be annealed to obtain any necessary phase separation at, near or above the A-block melting temperature, Tₘ. In addition, or in the alternative, the resulting adhesive may be sterilized, such as by treating it with ethylene oxide at a high temperature.

In illustrative examples, the tackifying agent may comprise a substantially amorphous biodegradable, bioabsorbable polymer with a molecular weight below the entanglement molecular weight. In addition, or alternative, the tackifying agent may have a glass transition above about 0 °C, more particularly above about 20 °C. In some examples, the tackifying agent may comprise a random copolymer of poly(L-lactide)-co-polyglycolide (PLGA) having a molecular weight of from about 1 to about 8 kDA, more particularly from about 1.5 to about 5 kDa.

In illustrative examples, an extrudable hot melt adhesive comprises ratios of polymer A-B-A and tackifying agent such that the glass transition of the blend ranges from about -5°C to about 15°C, more particularly from 0°C to about 10 °C. In addition, or in the alternative, the rheological properties of the polymer such as bulk moduli need to be such that the polymer can flow to conform to the surface topography of deck (73) or underside (65), while at the same time, providing enough integrity to maintain cohesive strength and resisting shearing off and/or peeling off of end effector (40).

### B. Exemplary Humidity Tolerant Adhesives with A-B-C Block Terpolymer Configurations

In some instances, the humidity tolerant adhesive materials (e.g., one or more of layers (104, 204, 304, 114)) for a buttress body (102, 202, 302, 112) comprise polymers having a general A-B-C block terpolymer configuration, in which the C polymer block comprises a hydrophilic polymer. Generally it is theorized, but in no way limits the scope of this invention, that humidity tolerant adhesives comprising hydrophilic polymers may have better wet surface retention characteristics than adhesives comprising only hydrophobic polymers.

In some versions, the A-B-C block terpolymers may be combined with a water sorbent. Useful water sorbents may be selected from the group consisting of: carboxymethyl cellulose (CMC); polyvinylpyrrolidine (PVP); gelatin; hyaluronan; and combinations thereof. In some such examples, the A-B-C block terpolymers may be combined with water sorbent such that the resulting mixture comprises by its weight percentage from about 1% to about 60%, preferably from about 20% to about 40%, of the water sorbent.

The A polymer blocks are biodegradable, bioabsorbable, non-elastic, highly crystalline segments, which are homopolymers that may be characterized by a relatively high glass transition temperature (Tg) and/or a relatively high crystallinity. In illustrative examples, the A homopolymers may be characterized by a glass transition temperature (Tg) of at least about 0°C, more particularly at least about 21°C (i.e., room temperature). In addition, or in the alternative, the A homopolymers may be characterized by a crystallinity as measured by X-ray diffraction of at least about 30%, more particularly at least about 40%, or more particularly at least about 45%. In addition, or in the alternative, the A homopolymers may have a molecular weight of at least about 5 kDa. Such exemplary A homopolymers may further be characterized by a melting temperature (Tm) of at least about 50°C, more particularly at least about 60°C, and more particularly at least about 70°C.

Exemplary A homopolymers may be selected from the group of: poly(L-lactide) (PLLA); poly(caprolactone) (PCL); polyglycolide (PGA); poly(3-hydroxybutyrate) (PH₃B); poly(3-hydroxyvalerate) (PHV); and poly(p-dioxanone) (PPDO). It may be difficult to synthesize 100% A homopolymers. The A homopolymers may thus contain a small percentage of residual B monomers. For example, exemplary A homopolymers may contain a small percentage (e.g., up to about 10% by weight) of B monomers.

The B polymer blocks are biodegradable, bioabsorbable, elastomeric homopolymers or co-polymers, which are predominantly amorphous and may be characterized by a relatively low to moderate glass transition temperature (Tg). In illustrative examples, the B polymers as homopolymers or co-polymers may be characterized by glass transition temperature (Tg) of at least about -40°C, more particularly at least about -30°C, and more particularly at least about -20 °C. In addition, or in the alternative, the B homopolymers or co-polymers may be characterized by a crystallinity as measured by X-ray diffraction of at most about 25%, more particularly at most about 10%, or more particularly at most about 5%. In addition, or in the alternative, the B polymers as homopolymers or co-polymers may have a molecular weight of from about 20 to about 80 kDa, more particularly from about 30 to about 70 kDa, and more particularly, from about 40 to about 65 kDa. In addition, or in the alternative, the B polymers as homopolymers may have an entanglement molecular weight of from about 3 to 4 kDa.

Exemplary B homopolymers or co-polymers comprise monomers selected from the group consisting of: caprolactone (CL), L-Lactide (LLA), D,L-Lactide ((D,L)LA), Glycolide (GA), Polydioxanone (PDO), Trimethylene carbonate (TMC), sebacic acid (SA), 1,6-bis(carboxyphenoxy)hexane (CPH), and combinations thereof. As another merely illustrative example, B polymers or co-polymers may be selected from the group of: caprolactone-co-glycolide (CAP-co-GLY); poly(L-lactide)-co-glycolide (PLGA); poly(D,L-lactide) (P(D,L)LA); poly(caprolactone)-co-glycolide (PCL-co-GA); poly[(1,6-bis(p-carboxyphenoxy)hexane)-co-sebacic acid (PCPH-co-SA); poly(trimethylene carbonate) (PTMC); poly(trimethylene carbonate)-co-glycolide (PTMC-co-GA; and poly(trimethylene carbonate)-co-caprolactone (PTMC-co-CL).

The C polymer blocks are biodegradable, bioabsorbable, hydrophilic homopolymers or co-polymers and may be characterized by miscibility with water at 37°C. Exemplary C homopolymers and co-polymers may be selected from the group of: polyethylene oxide (PEG); polyethylene oxide-co-polypropylene oxide (PEO-co-PPO); polyethylene oxide-co-polysulfone (PEO-co-PSO); polyvinylpyrrolidine (PVP); polyacrylic acid (PAA); and polyvinyl alcohol (PVOH).

In an illustrative example of a useful A-B-C block terpolymer, A is glycolide (GLY), B is a co-polymer of caprolactone-glycolide (CAP-co-GLY) and C is polyethylene oxide (PEO).

### V. U.S. Pat. App. No. 14/926,057 describes:

### A. Adhesion of Buttress to Wet End Effector Using Humidity Tolerant Adhesive Materials

In some surgical applications, it may be desirable to provide a buttress body (102, 112) with one or more humidity tolerant adhesive materials that will at least temporarily adhere to a wet end effector (40), particularly when it is being used intraoperatively. In some instances, humidity tolerant adhesive materials may provide for temporary attachment of a buttress body (102, 112) to the wet deck (73) of staple cartridge (70) or the wet underside (65) of anvil (60). A humidity tolerant adhesive material is defined herein as an adhesive material that holds a buttress body (102, 112) in place on an anvil (60) or staple cartridge (70) for at least five minutes in an environment of 100% relative humidity (e.g., in a patient's body, at a normal body temperature of approximately 37°C), preferably after the buttress body (102, 112) has been exposed to a relative humidity of from about 20% to about 60% for up to one hour at room temperature (e.g., between approximately 20°C and approximately 22°C). In some instances, a humidity tolerant adhesive material may hold a buttress body (102, 112) in place on an anvil (60) or staple cartridge (70) for at least ten minutes in an environment of 100% relative humidity (e.g., in a patient's body, at a normal body temperature of approximately 37°C), preferably after the buttress body (102, 112) has been exposed to a relative humidity of from about 20% to about 60% for up to one hour at room temperature (e.g., between approximately 20°C and approximately 22°C). A pressure sensitive humidity tolerant adhesive material is defined herein as a humidity tolerant adhesive material that can be transferred from a delivery device onto an anvil (60) or staple cartridge (70) by the pressure respectively exerted by the anvil (60) or staple cartridge (70).

Adhesive layers (104, 114) respectively provide for temporary attachment of the buttress bodies (102, 112) to underside (65) of anvil (60) and deck (73) of staple cartridge (70). It should be understood that the humidity tolerant adhesive material need not necessarily constitute a separate adhesive layer (104, 114) that is discretely identifiable as being different from a layer defined by buttress body (102, 112). Examples of humidity tolerant adhesive materials that may be otherwise integrated onto or into a buttress body (102, 112) are described in further detail below.

In some instances, the humidity tolerant adhesive materials (e.g., one or more of layers (104, 114)) for a buttress body (102, 112), comprise polymers that are either bioabsorbable or of a molecular weight that is sufficiently low so as to be cleared from the patient's body (e.g., less than approximately 30,000 KDa). Various physiomechanical properties of polymers may be modified in order to provide different adhesive properties. Such variable characteristics include but are not limited to the following: copolymer composition; copolymer architecture (e.g., random vs. block configurations, polymer branching, etc.); glass transition temperature (Tg); molecular weight; crystallinity; sequence distribution; copolymer chain composition; melting temperature (Tm); solubility or dissolution rate; rheological properties; surface tension; and combinations thereof. Several exemplary combinations of these variables will be provided below, though it should be understood that these examples are merely illustrative.

In addition or in the alternative to the aforementioned modifications to the physiomechanical properties of polymers, some exemplary humidity tolerant adhesive materials may comprise polymers that are combined with sorbents. Useful sorbents may be selected from the group consisting of: polysaccharides such as cellulose; cellulose derivatives, e.g., sodium carboxymethylcellulose (Na-CMC); starch; starch derivates; natural gums, e.g., agar and alginates; chitosan; pectin; gelatin; and combinations thereof. In some examples, a hydrocolloid of one or more sorbents may be mixed with the polymers. In some examples, the humidity tolerant adhesive material comprises a blend of sorbent and polymer in a ratio in a range of 70:30 sorbent to polymer, more particularly in a range of 50:50 sorbent to polymer, more preferably in a range of 10:90 sorbent to polymer. Generally it is theorized, but in no way limits the scope of this invention, that sorbents may act to absorb moisture away from the surface interface between the humidity tolerant adhesive material and the surface to which it is adhered (e.g., a wet end effector (40)), and to maintain the adherence of the buttress body (102, 112) to said surface until such time as the buttress body (102, 112) is deployed or released from end effector (40).

One of the aforementioned physiomechanical properties of polymers is glass transition temperature (Tg). Glass transition temperature is the temperature at which the mechanical properties of a copolymer change dramatically from a flowable adhesive to a brittle plastic. It may thus be of importance that the glass transition temperature (Tg) is sufficiently below the operating temperature of the humidity tolerant adhesive material in order to allow for sufficient polymer chain mobility. The melting temperature (Tm) of a polymer may be referred to as the "first-order transition," which is where the polymer changes state from solid to liquid. Crystalline polymers have a true melting point, which is the temperature at which the crystallites melt and the total mass of plastic becomes liquid. Amorphous polymers do not have a true melting point, but they do have a first-order transition wherein their mechanical behavior transitions from a rubbery nature to viscous rubbery flow. Suitable polymers for use in humidity tolerant adhesive materials may be semi-crystalline, i.e., they may have both amorphous and crystalline segments. Suitable polymers may have a melting point that is sufficiently above the operating temperature of the humidity tolerant adhesive material to maintain cohesive strength and to provide dimensional stability of the applied humidity tolerant adhesive material.

The molecular weight of non-bioabsorbable polymers should be high enough to provide mechanical strength to the resulting adhesive material in order to avoid cohesive failure, yet low enough that they can be cleared by the patient's body. In the case of biodegradable polymers, an upper limit on molecular weight may not be required to provide polymer breakdown products are small enough to be cleared by the patient's body.

The solubility or dissolution rate of polymers in the aqueous environments that may be encountered during surgery depend upon a number of polymer characteristics including, but not limited to: polymer composition; polymer architecture; degree of cross-linking; block length; crystallinity; molecular weight; branching; and combinations thereof. In illustrative examples described below, certain polymers and co-polymers are chosen and combined with these characteristics in mind in order to decrease the dissolution rate of the resulting humidity tolerant adhesive materials that arc of use for adhering a buttress body (102, 112) to a wet end effector (40) during surgery, and maintaining the adherence of the buttress body to the wet end effector (40) until such time as the buttress body (102, 112) is deployed or released from end effector (40).

The surface tension and rheology of polymers present in a humidity tolerant adhesive material may also impact its adhesive properties. For example, if there is a sufficiently large mismatch between the surface tension of the polymers and the surfaces to which it will adhere, adhesion between the two may be energetically unfavorable. Similarly, the rheological properties of the polymer such as bulk modulus may be such that the humidity tolerant adhesive material can flow to conform to the surface topography of the end effector (40), while at the same time providing sufficient integrity to maintain cohesive strength and resist shearing and peeling of the buttress body (102, 112) from the end effector (40).

### 1. Exemplary Humidity Tolerant Adhesive Materials Comprising Poloxamer Blends

In some examples, the humidity tolerant adhesive materials (e.g., one or more of layers (104, 114)) for a buttress body (102, 112) may comprise a blend of "plastic fats", more particularly, poloxamers. In illustrative examples, the blend of poloxamers may comprise a blend of poloxamers selected from the group consisting of: poloxamer 188, for example Kolliphor^{®} P188 from BASF (Florham Park, NJ); Synperonic^{®} PE/P84 from Croda Inc. (Edison, NJ); poloxamer 124, for example Pluronic^{®} L44 from BASF (Florham Park, NJ); poloxamer 407, for example Pluronic^{®} F-127 from BASF (Florham Park, NJ); and combinations thereof. Preferably, the poloxamers are of National Formulary grade. The resulting poloxamer-based humidity tolerant adhesive materials may be putty-like materials with a relatively low crystallinity and low glass transition temperature (Tg). Generally it is theorized, but in no way limits the scope of this invention, that the presence of polypropylene oxide repeat units in the backbone of the poloxamers provides for a poloxamer blend having a slower dissolution rate, which may desirably provide for humidity tolerant adhesive materials having a greater humidity (i.e., wetness) tolerance. In turn, a buttress body (102, 112), to which poloxamer-based adhesive materials have been applied, may desirably remain adhered to a wet end effector (40) of a surgical stapling instrument (10) during a surgical procedure until such time as buttress body (102, 112) is deployed.

In some examples, the humidity tolerant adhesive materials comprise a poloxamer blend of poloxamer 188 and Synperonic^{®} PE/P84 in a molar ratio in the range of from 1:3 to 1:4 of poloxamer 188 to Synperonic^{®} PE/P84. In some other examples, humidity tolerant adhesive materials comprise a poloxamer blend of poloxamer 188 and poloxamer 124 in a molar ratio in the range of from about 1:1 to about 1:4, more particularly from about 1:1.5 to about 1:3, of poloxamer 188 to poloxamer 124. In yet some other examples, the poloxamer blend may comprise a blend of poloxamer 407 and poloxamer 124 in a molar ratio in the range of from about 1:1, to about 1:5, more particularly from about 1:1.5 to about 1:3 of poloxamer 407 to poloxamer 124.

In yet some other examples, the poloxamers may be combined with non-ionic surfactants to modify the hydrophobicity of the resulting humidity tolerant adhesive material. In some such examples, the poloxamers may be combined with non-ionic surfactants selected from the group consisting of: polysorbates; polyethylene glycol hexadecyl ether, for example Brij 52 from Croda Inc. (Edison, NJ); sorbitane monooleate, for example, Span^{®} 80 from Sigma Aldrich (Saint Louis, MO); and combinations thereof.

In each of the foregoing exemplary poloxamer blends, it may be important to control the crystallite size of the poloxamers in order to achieve the desirable adhesive characteristics in the resulting humidity tolerant adhesive material.

### 2. Exemplary Humidity Tolerant Adhesive Materials Comprising Polyethylene Glycol or Polyethylene-Polyethylene Glycol Co-polymer

In some examples, the humidity tolerant adhesive materials (e.g., one or more of layers (104, 114)) for a buttress body (102, 112) comprise polyethylene glycol (PEG) or polyethylene-polyethylene glycol co-polymers (PE-co-PEG). The resulting humidity tolerant adhesive materials may be putty-like, malleable and extrudable.

In yet further examples, the pressure sensitive humidity tolerant adhesive materials comprise, or consist essentially of, polyethylene-polyethylene glycol co-polymers (PE-co-PEG) with a molecular weight that is sufficiently low so as to be cleared from the patient's body (e.g., less than approximately 30,000 KDa).

In yet further examples, the humidity tolerant adhesive materials comprise a blend of polyethylene-polyethylene glycol copolymers (PE-co-PEG) and poly(caprolactone)-glycolide copolymers (PCL/PGA) in the ratio of about 40:60 PCL:PGA, preferably in a ratio of about 50:50 PCL:PGA, more preferably in a ratio of about 60:40 PCL:PGA. Such a blend may have low crystallinity and may even be near amorphous.

In yet further examples, the humidity tolerant adhesive materials comprise a blend of polyethylene glycol having different molecular weights that is in turn blended with a polymer or co-polymer selected from the group consisting of: poloxamers; poly(caprolactone)-glycolide copolymers (PCL/PGA); lactide (PLA); and combinations thereof. By way of example only, the blend may include polyethylene glycol 3350 (PEG 3350), polyethylene glycol 400 (PEG 400), and/or other polyethylene glycols.

In yet further examples, the humidity tolerant adhesive materials comprise a block copolymer of polyethylene glycol 20,000 (PEG 20,000) and poly(caprolactone)-glycolide copolymers (PCL/PGA) that are characterized by a molarratio of 65:35 poly(caprolactone) (PCL) to glycolide (PGA). The resulting blends may have a relatively high molecular weight and lower solubility.

As yet another merely illustrative example, the humidity tolerant adhesive materials comprise a blend of other water soluble copolymers with poloxamers or PEG, with a molecular weight low enough to be cleared from the patient's body. Such a blend may be substituted for a component of any of the blends described above; or for the entirety of any of the blends described above. By way of further example only, the polymer(s) in such a blend may be biodegradable such as PCL/PGA, etc.

### 3. Exemplary Humidity Tolerant Adhesive Materials Comprising Solid Triglycerides in Oil

In some examples, the humidity tolerant adhesive materials (e.g., one or more of layers (104, 114)) for a buttress body (102, 112) comprise "plastic fats" comprising solid triglycerides in oil. In some illustrative examples, such humidity tolerant adhesive materials further comprise sorbents. Useful sorbents may be selected from the group consisting of: polysaccharides such as cellulose; cellulose derivatives, e.g., sodium carboxymethylcellulose (Na-CMC); starch; starch derivates; natural gums, e.g., agar and alginates; chitosan; pectin; gelatin; and combinations thereof. Useful triglycerides may be selected from the group consisting of: decanoyl glycerides; octanoyl glycerides; and combinations thereof-for example, Miglyol^{®} 810, 812, 818 and 829 from Caesar & Loretz GMBH (Hilden, DE). Useful oils may be selected from the group consisting of: bis-diglyceryl polyacyladipate-1; glycerol trioheptanoate; and combinations thereof-for example, Softisan^{®} 645 and Spezialöl 107 from Cremer Care (Hamburg, GE). The resulting humidity tolerant adhesive materials may desirably provide for good adhesion to end effector (40) and good spreading properties.

### 4. Exemplary humidity Tolerant Adhesive Materials Comprising Hydrocolloid Gels

In some examples, the humidity tolerant adhesive materials (e.g., one or more of layers (104, 114)) for a buttress body (102, 112) comprise hydrocolloid gels. In some illustrative examples, useful hydrocolloid gels may be selected from the group consisting of gels comprising: chitosan; carboxymethyl cellulose (CMC); ethyl cellulose; hydroxypropylmethyl cellulose; gelatin; and combinations thereof. The resulting humidity tolerant adhesive materials may have a relatively high water binding capacity.

### VI. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

In addition to the foregoing, it should also be understood that any of the various buttress assemblies described herein may be further constructed and operable in accordance with at least some of the teachings of U.S. Patent App. No. 14/667,842, entitled "Method of Applying a Buttress to a Surgical Stapler," filed March 25, 2015, the disclosure of which is referenced herein; U.S. Patent App. No. 14/827,856, entitled "Implantable Layers for a Surgical Instrument," filed August 17, 2015, the disclosure of which is referenced herein; U.S. Patent App. No. 14/871,071, entitled "Compressible Adjunct with Crossing Spacer Fibers," filed September 30, 2015, the disclosure of which is referenced herein; and U.S. Patent App. No. 14/871,131, entitled "Method for Applying an Implantable Layer to a Fastener Cartridge," filed September 30, 2015, the disclosure of which is referenced herein. Furthermore, in addition to the methods described herein, any of the various buttress assemblies described herein may be applied to end effector (40) in accordance with at least some of the teachings of U.S. Provisional Patent App. No. 62/209,041, entitled "Method and Apparatus for Applying a Buttress to End Effector of a Surgical Stapler," filed August 24, 2015, the disclosure of which is referenced herein; and/or U.S. Patent App. No. 14/871,131, entitled "Method for Applying an Implantable Layer to a Fastener Cartridge," filed September 30, 2015, the disclosure of which is referenced herein. Various suitable ways in which the teachings herein may be combined with various teachings of the above-cited references will be apparent to those of ordinary skill in the art.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued August 11, 1998, the disclosure of which is referenced herein; U.S. Pat. No. 5,817,084, entitled "Remote Center Positioning Device with Flexible Drive," issued October 6, 1998, the disclosure of which is referenced herein; U.S. Pat. No. 5,878,193, entitled "Automated Endoscope System for Optimal Positioning," issued March 2, 1999, the disclosure of which is referenced herein; U.S. Pat. No. 6,231,565, entitled "Robotic Arm DLUS for Performing Surgical Tasks," issued May 15, 2001, the disclosure of which is referenced herein; U.S. Pat. No. 6,783,524, entitled "Robotic Surgical Tool with Ultrasound Cauterizing and Cutting Instrument," issued August 31, 2004, the disclosure of which is referenced herein; U.S. Pat. No. 6,364,888, entitled "Alignment of Master and Slave in a Minimally Invasive Surgical Apparatus," issued April 2, 2002, the disclosure of which is referenced herein; U.S. Pat. No. 7,524,320, entitled "Mechanical Actuator Interface System for Robotic Surgical Tools," issued April 28, 2009, the disclosure of which is referenced herein; U.S. Pat. No. 7,691,098, entitled "Platform Link Wrist Mechanism," issued April 6, 2010, the disclosure of which is referenced herein; U.S. Pat. No. 7,806,891, entitled "Repositioning and Reorientation of Master/Slave Relationship in Minimally Invasive Telesurgery," issued October 5, 2010, the disclosure of which is referenced herein; U.S. Pub. No. 2013/0012957, entitled "Automated End Effector Component Reloading System for Use with a Robotic System, published January 10, 2013, the disclosure of which is referenced herein; U.S. Pub. No. 2012/0199630, entitled "Robotically-Controlled Surgical Instrument with Force-Feedback Capabilities," published August 9, 2012, the disclosure of which is referenced herein; U.S. Pub. No. 2012/0132450, entitled "Shiftable Drive Interface for Robotically-Controlled Surgical Tool," published May 31, 2012, the disclosure of which is referenced herein; U.S. Pub. No. 2012/0199633, entitled "Surgical Stapling Instruments with Cam-Driven Staple Deployment Arrangements," published August 9, 2012, the disclosure of which is referenced herein; U.S. Pub. No. 2012/0199631, entitled "Robotically-Controlled Motorized Surgical End Effector System with Rotary Actuated Closure Systems Having Variable Actuation Speeds," published August 9, 2012, the disclosure of which is referenced herein; U.S. Pub. No. 2012/0199632, entitled "Robotically-Controlled Surgical Instrument with Selectively Articulatable End Effector," published August 9, 2012, the disclosure of which is referenced herein; U.S. Pub. No. 2012/0203247, entitled "Robotically-Controlled Surgical End Effector System," published August 9, 2012, the disclosure of which is referenced herein; U.S. Pub. No. 2012/0211546, entitled "Drive Interface for Operably Coupling a Manipulatable Surgical Tool to a Robot," published August 23, 2012; U.S. Pub. No. 2012/0138660, entitled "Robotically-Controlled Cable-Based Surgical End Effectors," published June 7, 2012, the disclosure of which is referenced herein; and/or U.S. Pub. No. 2012/0205421, entitled "Robotically-Controlled Surgical End Effector System with Rotary Actuated Closure Systems," published August 16, 2012, the disclosure of which is referenced herein.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical stapler end effector assembly, the end effector assembly comprising:
(a) a staple cartridge (70), wherein the staple cartridge comprises:
(i) a plurality of staples (90), and
(ii) a deck (73), wherein the staple cartridge is operable to drive the staples through the deck;
(b) an anvil (60), wherein the anvil is movable from an open position toward the staple cartridge to reach a closed position, wherein the anvil includes an underside (65) having a staple forming surface configured to receive staples driven through the deck; and
(c) a buttress assembly (200, 300), wherein the buttress assembly is sized and configured to be coupled with the deck of the staple cartridge or the underside of the anvil, wherein the buttress assembly comprises:
(i) a buttress body (202, 302),
(ii) a first adhesive layer (204, 304) laid over the buttress body, wherein the first adhesive layer is formed of a first adhesive material, and
(iii) a second adhesive layer (206, 306) laid over the first adhesive layer, such that the first adhesive layer is interposed between the second adhesive layer and the buttress body, wherein the second adhesive layer is formed of a second adhesive material, wherein the second adhesive material is different from the first adhesive material,
wherein the second adhesive material comprises regions (308) contacting the buttress body.

2. The end effector assembly of claim 1, wherein the second adhesive layer is positioned to face one or more of: the underside of the anvil; and the deck of the staple cartridge.

3. The end effector assembly of claim 1 or claim 2, wherein the first adhesive material has one or more of: greater pliability than the second adhesive material; and greater tackiness than the second adhesive material.

4. The end effector assembly of any one of claims 1 to 3, wherein the second adhesive material is one or more of: more resistant to moisture than the first adhesive material; and more resistant to temperature than the first adhesive material.

5. The end effector assembly of any one of claims 1 to 4, wherein the first and second adhesive layers are configured to cooperate to adhere the buttress body to either the deck of the staple cartridge or the underside of the anvil.

6. The end effector assembly of any one of claims 1 to 5, wherein the first and second adhesive materials have one or more of: different molecular weight blends; and different cross-linking properties.

7. The end effector assembly of any one of claims 1 to 6, wherein the first and second adhesive materials are selected from different families of adhesive materials.

8. The end effector assembly of any one of claims 1-7, wherein the buttress body and the regions of the second adhesive material contacting the buttress body cooperate to encapsulate the first adhesive material.

9. The end effector assembly of claim 1 or 8, wherein the first adhesive layer has a length, wherein the regions of the second adhesive material contacting the buttress body extend along the full length of the first adhesive layer.

10. The end effector assembly of any one of claims 1 to 9, wherein the first adhesive layer has a width, wherein the regions of the second adhesive material contacting the buttress body extend along the full width of the first adhesive layer.

11. The end effector assembly of any one of claims 1 to 10, wherein the buttress assembly is adhered to the underside of the anvil, wherein the staples are positioned to be driven through the buttress assembly.

12. A method of making a buttress assembly for use with a surgical stapler end effector, wherein the end effector comprises a staple cartridge and an anvil, wherein the staple cartridge comprises a plurality of staples and a deck, wherein the staple cartridge is operable to drive the staples through the deck, wherein the anvil is movable from an open position toward the staple cartridge to reach a closed position, wherein the anvil includes an underside having a staple forming surface configured to receive staples driven through the deck, the method comprising:
(a) applying a first adhesive layer to a buttress body, wherein the buttress body is sized and configured to fit on the deck of the staple cartridge or the underside of the anvil, wherein the first adhesive layer comprises a first adhesive material; and
(b) applying a second adhesive layer to the first adhesive layer,
such that the first adhesive layer is interposed between the second adhesive layer and the buttress body, wherein the second adhesive layer is formed of a second adhesive material, wherein the second adhesive material is different from the first adhesive material, wherein the second adhesive material comprises regions (308) contacting the buttress body.

13. The method of claim 12, wherein the act of applying the second adhesive layer to the first adhesive layer comprises spraying the second adhesive layer over the first adhesive layer.

14. The method of claim 12 or claim 13, further comprising pressing the buttress assembly against the underside of the anvil, wherein one or both of the first or second adhesive materials secures the buttress assembly to the underside of the anvil in response to pressing the buttress assembly against the underside of the anvil.

## Patentansprüche

1. Endeffektoranordnung für eine chirurgisches Klammervorrichtung, die Endeffektoranordnung umfassend:
(a) ein Klammermagazin (70), wobei das Klammermagazin umfasst:
(i) eine Vielzahl von Klammern (90) und
(ii) eine Ladefläche (73), wobei das Klammermagazin betriebsfähig ist, um die Klammern durch die Ladefläche hindurch zu treiben;
(b) einen Amboss (60), wobei der Amboss aus einer offenen Position zu dem Klammermagazin hin bewegbar ist, um eine geschlossene Position zu erreichen, wobei der Amboss eine Unterseite (65) einschließt, die eine Klammerausbildungsoberfläche aufweist, die konfiguriert ist, um Klammern aufzunehmen, die durch die Ladefläche hindurch getrieben werden; und
(c) eine Stützpfeileranordnung (200, 300), wobei die Stützpfeileranordnung bemessen und konfiguriert ist, um mit der Ladefläche des Klammermagazins oder der Unterseite des Ambosses gekoppelt zu werden, wobei die Stützpfeileranordnung umfasst:
(i) einen Stützpfeilerkörper (202, 302),
(ii) eine erste Klebeschicht (204, 304), die über den Stützpfeilerkörper gelegt ist, wobei die erste Klebeschicht aus einem ersten Klebematerial ausgebildet ist, und
(iii) eine zweite Klebeschicht (206, 306), die über der ersten Klebeschicht derart gelegt ist, dass die erste Klebeschicht zwischen der zweiten Klebeschicht und dem Stützpfeilerkörper eingefügt ist, wobei die zweite Klebeschicht aus einem zweiten Klebematerial ausgebildet ist, wobei sich das zweite Klebematerial von dem ersten Klebematerial unterscheidet,
wobei das zweite Klebematerial Bereiche (308) umfasst, die den Stützpfeilerkörper berühren.

2. Endeffektoranordnung nach Anspruch 1, wobei die zweite Klebeschicht positioniert ist, um zu einem oder mehreren gerichtet zu sein von: der Unterseite des Ambosses; und der Ladefläche des Klammermagazins.

3. Endeffektoranordnung nach Anspruch 1 oder 2, wobei das erste Klebematerial eines oder mehrere aufweist von: einer höheren Biegsamkeit als das zweite Klebematerial; und einer höheren Klebrigkeit als das zweite Klebematerial.

4. Endeffektoranordnung nach einem der Ansprüche 1 bis 3, wobei das zweite Klebematerial eines oder mehrere ist von: feuchtigkeitsbeständiger als das erste Klebematerial; und temperaturbeständiger als das erste Klebematerial.

5. Endeffektoranordnung nach einem der Ansprüche 1 bis 4, wobei die erste und die zweite Klebeschicht konfiguriert sind, um zusammenzuwirken, um den Stützpfeilerkörper entweder an der Ladefläche des Klammermagazins oder der Unterseite des Ambosses zu kleben.

6. Endeffektoranordnung nach einem der Ansprüche 1 bis 5, wobei das erste und das zweite Klebematerial eines oder mehrere aufweisen von: Mischungen mit unterschiedlichem Molekulargewicht; und unterschiedlichen Vernetzungseigenschaften.

7. Endeffektoranordnung nach einem der Ansprüche 1 bis 6, wobei das erste und das zweite Klebematerial aus unterschiedlichen Klebematerialfamilien ausgewählt sind.

8. Endeffektoranordnung nach einem der Ansprüche 1 bis 7, wobei der Stützpfeilerkörper und die Bereiche des zweiten Klebematerials, die den Stützpfeilerkörper berühren, zusammenwirken, um das erste Klebematerial einzukapseln.

9. Endeffektoranordnung nach Anspruch 1 oder 8, wobei die erste Klebeschicht eine Länge aufweist, wobei die Bereiche des zweiten Klebematerials, die den Stützpfeilerkörper berühren, sich über die gesamte Länge der ersten Klebeschicht erstrecken.

10. Endeffektoranordnung nach einem der Ansprüche 1 bis 9, wobei die erste Klebeschicht eine Breite aufweist, wobei die Bereiche des zweiten Klebematerials, die den Stützpfeilerkörper berühren, sich über die gesamte Breite der ersten Klebeschicht erstrecken.

11. Endeffektoranordnung nach einem der Ansprüche 1 bis 10, wobei die Stützpfeileranordnung an der Unterseite des Ambosses geklebt ist, wobei die Klammern positioniert sind, um durch die Stützpfeileranordnung hindurch getrieben zu werden.

12. Verfahren zum Herstellen einer Stützpfeileranordnung zur Verwendung mit einem Endeffektor für eine chirurgische Klammervorrichtung, wobei der Endeffektor ein Klammermagazin und einen Amboss umfasst, wobei das Klammermagazin eine Vielzahl von Klammern und eine Lagerfläche umfasst, wobei das Klammermagazin betriebsfähig ist, die Klammern durch die Lagerfläche hindurch zu treiben, wobei der Amboss aus einer offenen Position zu dem Klammermagazin hin bewegbar ist, um eine geschlossene Position zu erreichen, wobei der Amboss eine Unterseite einschließt, die eine Klammerausbildungsoberfläche aufweist, die konfiguriert ist, um Klammern aufzunehmen, die durch die Lagerfläche hindurch getrieben werden, das Verfahren umfassend:
(a) Aufbringen einer ersten Klebeschicht auf einen Stützpfeilerkörper, wobei der Stützpfeilerkörper bemessen und beschaffen ist, um auf die Lagerfläche des Klammermagazins oder die Unterseite des Ambosses zu passen, wobei die erste Klebeschicht ein erstes Klebematerial umfasst; und
(b) Aufbringen einer zweiten Klebeschicht auf die erste Klebeschicht derart, dass die erste Klebeschicht zwischen der zweiten Klebeschicht und dem Stützpfeilerkörper eingefügt ist, wobei die zweite Klebeschicht aus einem zweiten Klebematerial ausgebildet ist, wobei sich das zweite Klebematerial von dem ersten Klebematerial unterscheidet, wobei das zweite Klebematerial Bereiche (308) umfasst, die den Stützpfeilerkörper berühren.

13. Verfahren nach Anspruch 12, wobei der Vorgang des Aufbringens der zweiten Klebeschicht auf die erste Klebeschicht ein Sprühen der zweiten Klebeschicht über die erste Klebeschicht umfasst.

14. Verfahren nach Anspruch 12 oder 13, ferner umfassend ein Pressen der Stützpfeileranordnung gegen die Unterseite des Ambosses, wobei eines oder beide der ersten und der zweiten Klebematerialien die Stützpfeileranordnung an der Unterseite des Ambosses als Reaktion auf das Pressen der Stützpfeileranordnung gegen die Unterseite des Ambosses befestigen.

## Revendications

1. Ensemble effecteur terminal d'agrafeuse chirurgicale, l'ensemble effecteur comprenant :
(a) une cartouche d'agrafes (70), dans lequel la cartouche d'agrafes comprend :
(i) une pluralité d'agrafes (90), et
(ii) un plateau (73), dans lequel la cartouche d'agrafes fonctionne pour actionner les agrafes à travers le plateau ;
(b) une enclume (60), dans lequel l'enclume peut être déplacée d'une position ouverte vers la cartouche d'agrafes pour atteindre une position fermée, dans lequel l'enclume comporte une face inférieure (65) présentant une surface de formation d'agrafes conçue pour recevoir des agrafes actionnées à travers le plateau ; et
(c) un ensemble contrefort (200, 300), dans lequel l'ensemble contrefort est dimensionné et conçu pour être accouplé au plateau de la cartouche d'agrafes ou à la face inférieure de l'enclume, dans lequel l'ensemble contrefort comprend :
(i) un corps de contrefort (202, 302),
(ii) une première couche adhésive (204, 304) posée sur le corps du contrefort, la première couche adhésive étant formée d'un premier matériau adhésif, et
(iii) une seconde couche adhésive (206, 306) posée sur la première couche adhésive, de telle sorte que la première couche adhésive est interposée entre la seconde couche adhésive et le corps de contrefort, la seconde couche adhésive étant formée d'un second matériau adhésif, le second matériau adhésif étant différent du premier matériau adhésif,
dans lequel le second matériau adhésif comprend des zones (308) en contact avec le corps de contrefort.

2. Ensemble effecteur terminal selon la revendication 1, dans lequel la seconde couche adhésive est positionnée de manière à faire face à un ou plusieurs parmi : la face inférieure de l'enclume ; et le plateau de la cartouche d'agrafes.

3. Ensemble effecteur terminal selon la revendication 1 ou la revendication 2, dans lequel le premier matériau adhésif présente une ou plusieurs parmi : une plus grande souplesse que le second matériau adhésif ; et une plus grande adhésivité que le second matériau adhésif.

4. Ensemble effecteur terminal selon l'une quelconque des revendications 1 à 3, dans lequel le second matériau adhésif est un ou plusieurs parmi : plus résistant à l'humidité que le premier matériau adhésif ; et plus résistant à la température que le premier matériau adhésif.

5. Ensemble effecteur terminal selon l'une quelconque des revendications 1 à 4, dans lequel la première et la seconde couche adhésive sont conçues pour coopérer afin de faire adhérer le corps de contrefort soit au plateau de la cartouche d'agrafes, soit à la face inférieure de l'enclume.

6. Ensemble effecteur terminal selon l'une quelconque des revendications 1 à 5, dans lequel les premier et second matériaux adhésifs présentent un ou plusieurs parmi : des mélanges de poids moléculaires différents ; et différentes propriétés de réticulation.

7. Ensemble effecteur terminal selon l'une quelconque des revendications 1 à 6, dans lequel les premier et second matériaux adhésif sont choisis parmi différentes familles de matériaux adhésifs.

8. Ensemble effecteur terminal selon l'une quelconque des revendications 1 à 7, dans lequel le corps de contrefort et les zones du second matériau adhésif en contact avec le corps de contrefort coopèrent pour encapsuler le premier matériau adhésif.

9. Ensemble effecteur terminal selon la revendication 1 ou 8, dans lequel la première couche adhésive présente une longueur, dans lequel les zones du second matériau adhésif en contact avec le corps de contrefort s'étendent sur toute la longueur de la première couche adhésive.

10. Ensemble effecteur terminal selon l'une quelconque des revendications 1 à 9, dans lequel la première couche adhésive présente une largeur, dans lequel les zones du second matériau adhésif en contact avec le corps de contrefort s'étendent sur toute la largeur de la première couche adhésive.

11. Ensemble effecteur terminal selon l'une quelconque des revendications 1 à 10, dans lequel l'ensemble contrefort est collé à la face inférieure de l'enclume, dans lequel les agrafes sont positionnées pour être actionnées à travers l'ensemble contrefort.

12. Procédé de fabrication d'un ensemble contrefort destiné à être utilisé avec un effecteur terminal d'agrafeuse chirurgicale, dans lequel l'effecteur terminal comprend une cartouche d'agrafes et une enclume, dans lequel la cartouche d'agrafes comprend une pluralité d'agrafes et un plateau, dans lequel la cartouche d'agrafes fonctionne pour actionner les agrafes à travers le plateau, dans lequel l'enclume est mobile d'une position ouverte vers la cartouche d'agrafes pour atteindre une position fermée, dans lequel l'enclume comporte une face inférieure présentant une surface de formation d'agrafes conçue pour recevoir les agrafes actionnées à travers le plateau, le procédé comprenant :
(a) l'application d'une première couche adhésive sur un corps de contrefort, dans lequel le corps de contrefort est dimensionné et conçu pour s'adapter au plateau de la cartouche d'agrafes ou au dessous de l'enclume, dans lequel la première couche adhésive comprend un premier matériau adhésif ; et
(b) l'application d'une seconde couche adhésive sur la première couche adhésive, de sorte que la première couche adhésive soit interposée entre la seconde couche adhésive et le corps du contrefort, la seconde couche adhésive étant formée d'un second matériau adhésif, le second matériau adhésif étant différent du premier matériau adhésif, le second matériau adhésif comportant des zones (308) en contact avec le corps du contrefort.

13. Procédé selon la revendication 12, dans lequel l'application de la seconde couche adhésive sur la première couche adhésive comprend la pulvérisation de la seconde couche adhésive sur la première couche adhésive.

14. Procédé selon la revendication 12 ou la revendication 13, comprenant en outre la pression de l'ensemble contrefort contre la face inférieure de l'enclume, dans lequel l'un ou les deux matériaux adhésifs (le premier ou le second) fixent l'ensemble contrefort à la face inférieure de l'enclume en réponse à la pression de l'ensemble contrefort contre la face inférieure de l'enclume.
